Europäisches Patentamt

European Patent Office

Office européen des brevets

(11) Numéro de publication: **0 008 245**
A1

# (12) DEMANDE DE BREVET EUROPEEN

(21) Numéro de dépôt: 79400421.8

(51) Int. Cl.³: **G 01 N 33/54**

(22) Date de dépôt: 26.06.79

(30) Priorité: 27.06.78 FR 7819126

(43) Date de publication de la demande: 20.02.80
Bulletin 80/4

(84) Etats contractants désignés: BE DE GB IT NL SE

(71) Demandeur: **Société Anonyme dite: SEBIA, 23 rue Maximilien Robespierre, F-92130 Issy-Les-Moulineaux (FR)**
Demandeur: **Pinon, Jean-Michel, Les Haies, F-51160 Germaine (FR)**
Demandeur: **Pinon, Geneviève, Les Haies, F-51160 Germaine (FR)**

(72) Inventeur: **Barouh, Guy, 23 rue Maximilien Robespierre, F-92130 Issy-Les-Moulineaux (FR)**
Inventeur: **Pinon, Jean-Michel, Les Haies, F-51160 Germaine (FR)**
Inventeur: **Pinon, Geneviève, Les Haies, F-51160 Germaine (FR)**

(74) Mandataire: **Bossard, Jacques René, 10 rue de la Pépinière, F-75008 Paris (FR)**

(54) **Système physico-biochimique pour la détection, le dosage et l'isolement de substances à activité antigénique.**

(57) Système physico-biochimique destiné à la détection, l'analyse qualitative et quantitative, l'isolement et la concentration de substances à activité antigénique contenues en solution et/ou en suspension dans un liquide, du type dans lequel ladite substance est immobilisée par voie biochimique sur un support physique par une réaction immunologique avec un réactif spécifique lié à ce support, caractérisé en ce que ledit support est constitué par une structure poreuse bidimensionnelle à travers laquelle ledit liquide peut circuler par filtration conformément à un circuit continu et éventuellement répétitif jusqu'à immobilisation pratiquement totale de ladite substance.

ACTORUM AG

-1-

*Système physico-biochimique pour la détection, le dosage et l'isolement de substances à activité antigènique*

*La présente invention est relative à un nouveau type de systèmes physico-biochimiques permettant aisément la détection, l'isolement et la concentration des substances antigéniques, en solution ou en suspension dans un liquide, à la préparation de ces systèmes et à leurs divers domaines d'applications en biologie et en médecine.*

*Les vingt dernières années ont vu se développer rapidement les techniques dites d'immunoélectrodiffusion, faisant intervenir essentiellement un processus d'électrosynérèse sur une membrane d'acétate de cellulose (voir en particulier PINON J.M., DROPSY G., Path.Biol.25, n°1,23-27 et la bibliographie qui suit).*

*On peut définir plus généralement ces techniques comme faisant appel à un système physico-biochimique pour la détection de substances à activité antigèniques contenues en solution et/ou en suspension dans un liquide, et dans lequel ladite substance est immobilisée par voie biochimique sur un support physique par une réaction immunologique avec un réactif spécifique lié à ce support, puis détectée par un réactif révélateur.*

*Par substance à activité antigènique, il est bien entendu que l'on entend tout substance antigène (ou haptène) ou anticorps prenant part à la formation d'immunocomplexes spécifiques.*

*Par réactif spécifique, on entend le composant du couple antigène/anticorps autre que celui à analyser. Ainsi, en d'autres termes, le réactif spécifique peut être un anticorps spé-*

cifique de la substance à analyser dans le cas où celle-ci ne possède pas d'activité anticorps et dans le cas où elle possède une activité anticorps, le réactif spécifique peut être soit un antigène spécifique de l'anticorps, soit un anticorps anti-globuline spécifique d'espèce.

Enfin, le support physique était jusqu'à présent la plupart du temps une membrane de gel d'agarose ou d'acétate de cellulose.

Malgré leur très grand intérêt dans les méthodes diagnostiques, ces systèmes étaient essentiellement statiques, c'est-à-dire installés dans des cuves d'électrophorèse recevant un volume déterminé du liquide à analyser. Il en résulte une double limitation du procédé sur le plan technique, dans le cas d'une dilution extrême de la substance à analyser dans le liquide;en effet la quantité qui sera présente dans la cuve sera infime; elle sera donc, à la limite, indétectable, soit qu'elle soit trop faible pour apparaître sous l'action du réactif révélateur, soit que la fraction prélevée dans la cuve ne contienne précisément pas de trace de la substance, trop disséminée au sein du grand volume total.

L'invention pallie cet inconvénient et en même temps,ouvre à cette technique des champs d'applications extrêmement vastes.

Dans son principe, l'invention consiste à créer un système du type précédent, mais dans lequel le support permet la circulation du liquide par filtration, conformément à un circuit continu, éventuellement répétitif,et à double sens.

L'invention vise donc un système physico-biochimique destiné à la détection, l'isolement et la concentration de substances à activité antigénique contenues en solution et/ou en suspension dans un liquide, du type dans lequel ladite substance est immobilisée par voie biochimique sur un support physique par une réaction immunologique avec un réactif spécifique lié à ce support, caractérisé en ce que ledit support est constitué par une structure poreuse bi-dimensionnelle à travers laquelle ledit liquide peut circuler par filtration conformément à un circuit continu et éventuellement répétitif jusqu'à immobilisation pratiquement totale de ladite substance. La mise en pratique de l'invention comporte plusieurs variantes, qui peuvent elles-mêmes faire l'objet de diverses combinaisons.

Selon une première variante, la structure bi-dimensionnelle est constituée par au moins une matière polymère, qui peut être d'origine naturelle (polysaccharides insolubles dans l'eau, tels que la gélose, l'agarose, les arabinanes, les mannanes, les galactanes, les xylanes etc... ou polyolosides tels la cellulose, l'amidon et leurs esters (acétate, nitrate...) éthers (méthylique, benzylique...) ainsi que leurs composés d'addition) ou d'origine synthétique :

- homopolymères tels ques les polystyrènes, les résines polyvinyliques ou polyacryliques, les polyoléfines, les polyhalooléfines, les polydiènes...

- hétéropolymères tels que les polyesters, les polyamides, les polyuréthanes, les polyimides, les polyanhydrides, les polycarbonates, les polyimines, les polysiloxanes etc...

Dans ce cas, la matière polymère peut se présenter sous forme d'une membrane continue, possèdant par elle-même une rigidité mécanique suffisante, ou déposée sur un support lui assurant cette rigidité, ou sous forme d'une couche mince de particules telles que des billes, soit associées à un substrat, comme enrobage de ce substrat, soit maintenues entre deux parois de ce substrat. Ce substrat peut lui-même être soit poreux quand la structure doit être traversée transversalement (verre fritté, tamis métalliques etc...), soit quelconque quand la structure est traversée latéralement, c'est-à-dire d'un champ à l'autre de la couche mince proprement dite.

La dimension des pores ou interstices est naturellement fonction de la nature des substances à analyser, étant entendu que le phénomène, de nature purement biochimique, n'a rien de commun avec une filtration macromoléculaire. En fait, le phénomène d'immobilisation sur ladite structure de la substance à analyser peut prendre au moins quatre aspects :

- Selon une première possibilité, le réactif spécifique devant intervenir dans cette immobilisation est fixé sur la structure de façon covalente. La structure ainsi "sensibilisée" joue alors le rôle d'immunoabsorbant pour la substance à analyser. La fixation covalente du réactif spécifique,de nature protéique et qui possède des résidus amines primaires, peut être réalisée au moyen de bromure de cyanogène selon la technique décrite par Cuatrecasas, sur une structure possèdant des radicaux hydroxyles libres. C'est le cas des polysaccharides insolubles dans l'eau,comme la cellulose ou ses dérivés. Si le réactif spécifique est de nature polysaccharidique , d'autres agents de couplage peuvent être utilisés, comme les diépoxydes. D'une façon générale, il y a intérêt à fixer la quantité maximum possible de réactif spécifique par unité de surface de façon à augmenter la capacité d'absorption de la membrane, à condition de conserver l'activité du réactif spécifique fixé et de ne pas modifier exagérément les caractéristiques physiques de la structure , par exemple son électro-endosmose et sa perméabilité. Dans ces conditions, on a intérêt à utiliser une préparation aussi purifiée que possible du réactif spécifique pour favoriser la fixation du matériel actif immunologiquement.

D'autres réactifs constituant des agents de couplage peuvent être utilisés , par exemple la trichlorotriazine.

- Selon une seconde possibilité, on peut utiliser un couplage hydrophobe comme décrit dans le brevet français 2.286.382.

- Selon une troisième possibilité,le réactif spécifique peut être initialement lié de façon non covalente à la structure. Dans ces conditions, l'immobilisation sur la structure peut être le résultat de la formation d'un immunoprécipité mettant en jeu les techniques d'immuno-précipitation ou de double diffusion.

Les structures en cellulose ou ses dérivés, esters par exemple, acétate de cellulose,esters mixtes tels qu'acétate et nitrate de cellulose ne donnent pas de phénomène d'absorption incompatible avec la migration des protéines. De telles structures se prêtent également au développement des immunoprécipités.

- Selon une quatrième possibilité, le réactif spécifique peut être associé à la structure par simple précipitation physique dans les pores ou interstices de celle-ci.

Cette quatrième possibilité s'applique précisément à la seconde variante de l'invention, selon laquelle la structure, au lieu d'être de nature polymérique, comme dans la première variante, est de nature minérale, telle que du verre fritté , un métal fritté ou poreux, céramique ou analogues; dans ce cas, ce matériau ne joue plus seulement le rôle de substrat pour la structure mais celui de la structure elle-même, qui reçoit par exemple par précipitation, le réactif spécifique , qui est fixé ou retenu sur lui par des forces purements physiques,pouvant être renforcées par des effets extérieurs, par exemple magnétiques. La suppression de cet appoint extérieur peut faciliter ensuite la libération du réactif et de la substance qu'il a fixé.

Les réactifs spécifiques utilisés peuvent être par exemple toute substance possèdant une activité immunologique, à savoir :
- toute substance antigènique (antigène ou anticorps),soit naturellement (animale ou végétale),soit synthétique,soit dé) rivée ou métabolisée.
- les polysaccharides,acides nucléiques et dérivés.
- les micro-organismes : virus,bactéries,parasites et mycoses.

La structure peut être sensibilisée en totalité ou en partie seulement avec un ou plusieurs réactifs de spécificité différente. Ainsi une même structure peut en différentes zones prédéterminées de sa surface, possèder des spécificités différentes et jouer le rôle d'immuno-absorbant multiple. Par exemple les réactifs spécifiques fixés par covalence en différentes zones de la structure peuvent être différentes : antigènes parasitaires, antigènes hydatiques,aspergillus,billarsien etc...

Les zones actives de la structure peuvent alors avoir des formes et des dispositions variées selon l'utilisation ultérieure de la membrane.Par exemple, les zones actives peuvent être des disques équirépartis,ou encore se présenter sous forme de bandes parallèles.

Une fois sensibilisée au moyen du réactif spécifique, la structure est équilibrée dans un milieu correspondant à celui où se font habituellement les réactions immunologiques , par exemple un tampon PBS pH 7,4. Il est avantageux d'ajouter une protéine n'affectant pas le procédé d'essai, par exemple de l'albumine bovine à la concentration de 1% qui aura pour rôle de diminuer le phénomène d'absorption non spécifique.

Si elle est trop fragile, la structure est dispoée sur un support poreux, par exemple un disque de verre fritté situé à la partie inférieure d'un récipient (par exemple cellule d'ultra-filtration, extrémité d'une seringue), qui peut recevoir la solution à analyser.

Cette solution est amenée au contact des zones immunoabsorbantes par passage  au travers de la structure, soit par simple gravité soit par une surpression délivrée par un gaz inerte comprimé au moyen d'un piston.

Il est également possible d'amener en contact la solution à analyser avec les zones  immunoabsorbantes en soumettant l'échantillon à une migration électrophorétique à la surface de la structure préalablement équilibrée dans un tampon convenable,par exemple un tampon Tris Véronal Fi 0,05 pH 8,6 de telle sorte que l'échantillon balaye toutes les zones immunoabsorbantes. Dans ce cas, les zones immunoabsorbantes seront disposées sous forme de bandes parallèles, perpendiculaires à la direction de migration.

Dans la pratique, et comme il a été évoqué au début,un avantage du système selon l'invention réside dans la possibilité de faire traverser la structure par des quantités en principe illimitées d'une solution contenant la substance à analyser. Ainsi,on peut accumuler sur la structure  l'intégralité des traces d'une telle substance contenues dans un très grand volume de la solution, ou recycler ladite solution un nombre de fois suffisant pour acquérir la certitude que toutes les traces qu'elle pouvait contenir ont bien été immobilisées.

On dispose ainsi d' un système permettant aussi bien la détection pure et simple de traces infimes, pratiquement indécelables par les moyens connus, d'une substance déterminée, que la concentration de cette substance à des fins d'analyse quantitative et même que l'isolement de cette substance à l'état purifié.

A cet effet, la substance à analyser étant immobilisée sur la structure, soit par immunoabsorption, soit par formation d'un immunoprécipité, la structure est lavée par une solution convenable pour éliminer les constituants de l'échantillon ou du réactif spécifique n'ayant pas participé à la réaction immunologique d'immobilisation. Cette solution de lavage peut être de l'eau physiologique tamponnée à un pH ne permettant pas la dissociation des immunocomplexes, par exemple pH 7,4. Elle peut contenir un agent tensio-actif tel que le tween 20 à la concentration 0,1%. Le lavage est effectué par circulation de la solution au travers de la structure par une légère surpression.

La mise en évidence de la substance à analyser est réalisée au moyen d'un réactif révélateur. Ce réactif révélateur peut être un antigène ou un anticorps marqué avec un groupement susceptible de fournir une indication analytique par exemple un groupement actif du point de vue enzymatique.

Le réactif révélateur doit pouvoir réagir spécifiquement avec la substance à analyser dans le cas où l'étape d'immobilisation a été obtenue par formation d'un immunoprécipité entre la substance à analyser et le réactif spécifique.

Le réactif révélateur est généralement un anticorps marqué par une enzyme par exemple la péroxydase, la glucoseoxydase, la phosphotase alcaline mais ce peut également être un antigène marqué par une enzyme. Le réactif révélateur est amené au contact des constituants avec lesquels il est susceptible de réagir par filtration au travers de la structure. Il peut être également recyclé plusieurs fois. Il est utilisé en solution aqueuse tamponnée, par exemple : PBS pH 7,4, qui peut avantageusement contenir une protéine n'interférant pas avec leprocédé d'essai par exemple de l'albumine bovine à la concentration de 1%. La concentration du réactif révélateur est telle que sur une structure témoin ne comportant aucun immunocomplexe,la révélation qu'il donne par l'intermédiaire de son groupement marqué,par exemple en développant une réaction colorée,est négligeable par rapport à la révélation qu'il donne au niveau des immunocomplexes immobilisés sur la structure sur laquelle il viendra se concentrer par réaction spécifique.

Dans ces conditions, l'étape de révélation peut être immédiatement consécutive à l'étape de fixation du réactif révélateur sur l'immunocomplexe.

On donne ci-après , à titre illustratif, le détail d'un certain nombre de techniques de mise en application de l'invention, étant entendu que ces exemples n'ont aucun caractère limitatif.

## EXEMPLE 1

Une membrane d'acétate de cellulose (Cellogel) de surface 50 $cm^2$ est placée dans un bécher contenant 60 ml $H_2O$. On ajoute sous agitation 1  de bromure de cyanogène finement broyé. Le pH est maintenu entre 10,2 et 11,2 par addition d'une solution de soude à 5%. La réaction est poursuivie 1/2 h à 4°C.La membrane est lavée par 3 bains de 5 mn chacun dans un tampon borate 0,1M pH 9,25. Elle est alors immergée pendant 4h et à 4°C dans 10 ml d'une solution d'anatoxine tétanique à 40 Lf/ml préparée en tampon borate 0,1M pH 9,25. Puis elle est immergée 1h dans le tampon borate contenant 0,1 M d'éthanolamine.La membrane est lavée en tampon phosphate 0,01 M + NaCl 0,15 M pH 7,4 (tampon PBS).

La membrane ainsi activée peut être conservée dans un sachet plastique scellé en milieu $H_2O$/glycérine 50/50 auquel on a ajouté 0,01 % de merthiolate.

Pour son utilisation, la membrane est éventuellement découpée et est disposée sur le verre fritté d'un Büchner monté sur fiole à vide. La membrane est traversée sous léger vide par 10 ml d'une solution d'un sérum humain dilué au 1/100 dans le tampon PBS auquel on a ajouté 1% d'albumine bovine. Le sérum est recyclé 2 fois.

La membrane est lavée par filtration de 50 ml de tampon PBS. La membrane est alors traversée par 10 ml d'une solution au 1/1000 d'un sérum de lapin anti IgG humain marqué à la péroxydase. La solution est recyclée 2 fois.

L'activité péroxydasique fixée sur la membrane par l'intermédiaire des anticorps antitétaniques présents dans le sérum étudié, est détectée par coloration à la benzidine. L'intensité de la coloration permet une appréciation de la quantité d'anticorps antitétaniques dans le sérum.

## EXEMPLE 2

Mêmesconditions que dans l'exemple 1 mais la membrane au lieu d'être immergée dans la solution d'anatoxine est sensibilisée en partie seulement en zone circulaire de 1cm de diamètre.Pour cela on dépose à sa surface un disque de 1cm de diamètre de papier Waterman n°3 imprégné de 50 µl de la solution d'anatoxine à 40 Lf/ml. Ceci permet d'avoir à proximité de la zone d'essai une zone témoin servant de référence.

## EXEMPLE 3

Mêmes conditions que dans l'exemple 1 mais la membrane au lieu d'être placée sur un Büchner est placée à la base d'une cellule d'ultrafiltration. La circulation des solutions est alors réalisée par une surpression d'azote.

La présente invention peut également avoir les application suivantes :
- immunologie en général
- immunologie hormonologique
- immunologie bactériologique et virologique
- immunologie parasitologique et mycologique
- immunologie hématologique
- immunologie histo-chimique
- immunologie pathologique

0008245

- *immunologie pharmacologique*
- *immunologie cancérologique*
- *immunologie vétérinaire*
- *préparations industrielles*
- *contrôle et fabrication dans l'industrie pharmaceutique.*

0008245

*Revendications de brevet*

1. *Système physico-biochimique destiné à la détection,l'analyse qualitative et quantitative,l'isolement et la concentration de substances à activité antigènique contenues en solution et/ou en suspension dans un liquide,du type dans lequel ladite substance est immobilisée par voie biochimique sur un support physique par une réaction immunologique avec un réactif spécifique lié à ce support, caractérisé en ce que ledit support est constitué par une structure poreuse bidimensionnelle à travers laquelle ledit liquide peut circuler par filtration conformément à un circuit continu et éventuellement répétitif jusqu'à immobilisation pratiquement totale de ladite substance.*

2. *Système selon la revendication 1, caractérisé en ce que ladite structure consiste en une membrane continue.*

3. *Système selon la revendication 1, caractérisé en ce que ladite structure consiste en une couche mince de particules fines juxtaposées.*

4. *Système selon l'une quelconque des revendications 1 à 3, caractérisé en ce que la structure est constituée par au moins une matière polymère choisie entre celles d'origine naturelle (polysaccharides insoblules dans l'eau tels la gélose, l'agarose, les arabinanes,les carraghenanes,les mannanes,les galactanes, les xylanes etc..., ou polyolosides tels la cellulose,l'amidon et leurs esters (acétate ,nitrate...) éthers(méthylique,benzylique...)ainsi que leurs composés d'addition),ou synthétique :*
   - *homopolymères tels que les polystyrènes,les résines polyvinyliques ou polyacryliques,les polyoléfines,les polyhalooléfines,les polydiènes etc...*
   - *hétéropolymères tels que les polyesters,les polyamides,les polyuréthanes,les polyimides ,les polyanhydrides,les polycarbonates,les polyimines,les polysiloxanes etc...*

5. *Système selon l'une quelconque des revendications 1 à 4, caractérisé en ce que la structure est associée à un substrat*

dont elle constitue un enrobage ou qui en forme les parois.

6. Système selon la revendication 5, caractérisée en ce que ledit substrat est lui-même poreux.

7. Système selon l'une quelconque des revendications 1 à 6, caractérisé en ce que le réactif spécifique est fixé sur la structure par des liaisons covalentes.

8. Système selon l'une quelconque des revendications 1 à 6, caractérisé en ce que le réactif spécifique est associé à la structure par précipitation physique.

9. Système selon l'une quelconque des revendications 1 à 8, caractérisé en ce que le réactif spécifique est choisi entre les substances antigèniques naturelles, synthétiques, leurs dérivés et leurs métabolites, les polysaccharides, les acides nucléiques et leurs dérivés, et les micro-organismes végétaux et animaux.

10. Système selon la revendication 9, caractérisé en ce que dans le but de diminuer le phénomène d'encombrement stérique, ledit réactif est fixé par l'agent de couplage par interposition de space-groups.

11. Système selon la revendication 9, caractérisé en ce que la structure est subdivisée en zones géométriques à spécificité différente, et ayant des formes et dispositions appropriées, à savoir cercles concentriques, bandes parallèles et analogues.

# DOCUMENTS CONSIDERES COMME PERTINENTS

| Catégorie | Citation du document avec indication, en cas de besoin, des parties pertinentes | Revendication concernée |
|---|---|---|
| X | FR - A - 2 283 903 (MILLIPORE CORP.)<br>* Revendications, exemple 7 * | 1,2,4, 5,6,9 |
| X | US - A - 3 793 445 (S.J. UPDIKE et al.)<br>* Revendications, colonne 3, lignes 1-5; colonne 2, lignes 70-72 * | 1 |
|  | FR - A - 2 307 268 (SUMMA CORP.)<br>* Revendications * | 1 |
| X | FR - A - 2 330 694 (H.P. GREGOR)<br>* Revendications; exemple 2 * | 1,2,4, 7,9 |
|  | DE - A - 2 339 111 (BATELLE)<br>* Revendications * | 1,2 |
| A | FR - A - 2 374 646 (F.X. COLE et al.)<br>* Revendications * | |
| A | GB - A - 1 148 574 (MOUN SINAI HOSPITAL RESEARCH)<br>* Revendications * | |

**CLASSEMENT DE LA DEMANDE (Int. Cl. ¹)**

G 01 N 33/54

**DOMAINES TECHNIQUES RECHERCHES (Int. Cl. ³)**

G 01 N 33/16
B 01 D 13/00

**CATEGORIE DES DOCUMENTS CITES**

X: particulièrement pertinent
A: arrière-plan technologique
O: divulgation non-écrite
P: document intercalaire
T: théorie ou principe à la base de l'invention
E: demande faisant interférence
D: document cité dans la demande
L: document cité pour d'autres raisons

&: membre de la même famille, document correspondant

Le présent rapport de recherche a été établi pour toutes les revendications

| Lieu de la recherche | Date d'achèvement de la recherche | Examinateur |
|---|---|---|
| La Haye | 09-10-1979 | VAN GOETHEM |

OEB Form 1503.1  06.78